# EUROPEAN PATENT APPLICATION

(11) **EP 3 295 982 A1**
(43) Date of publication of application: **21.03.2018**
(21) Application number: 17188277.2
(22) Date of filing: 29.08.2017
(51) Int. Cl.: A61M 5/32, A61M 5/34

(54) **INJECTION NEEDLE**

(30) Priority: 20.09.2016 JP 2016004583 U
(71) Applicant: Nanbu Plastics Co., Ltd., Shizuoka 421-0305 (JP)
(72) Inventor: Ozawa, Hiroshi, Shizuoka, 4210305 (JP); Inou, Akinori, Shizuoka, 4210305 (JP); Takahashi, Nobuyuki, Shizuoka, 4210305 (JP); Shinoda, Akihiro, Shizuoka, 4210305 (JP); Kani, Eiji, Shizuoka, 4210305 (JP)
(74) Representative: Uexküll & Stolberg

(57) **Abstract**

[Problem to be solved] To provide a novel and useful injection needle that allows design of the size of a needle base without being limited by the size of a syringe connecting part. [Means for solving the problem] As a hub, a needle base 11 that is formed to have a through hole into which a needle tube is inserted, and a tubular housing where both ends in the axial direction are opened and the needle base is fitted and stored in the inside of one end in the axial direction, are formed as separate injection molded parts, thereby allowing the needle base **11** to be largely designed. The housing and needle base 11 are joined by an adhesive that infiltrates from the needle tip side into a fitting gap **47** formed therebetween, or by welding. A needle tube shield part is in a box-lid shape, and the back side of a ceiling part thereof is provided with a tubular guiding part **69.** An edge surface **71** thereof abuts on the needle base **11,** and a side part **75** annularly holds and abuts on the housing. Accordingly, the needle base **11** does not move upon receiving the pressure of a drug solution.

## Description

### [Technical Field]

The present invention relates to an injection needle for transdermally administering a drug solution.

### [Background Art]

Injection has been used for a long time to administer drug solutions that cannot be orally administered. Drug solutions are often injected with one needle tube, and since administration by puncturing with an edge of a needle tube involves pain, reduction of pain has been recognized as an objective.

In this regard, from recent years, a type of injection needle that comprises a plurality of needle tubes is proposed in Patent Literature 1 and the like. By disposing a plurality of thin needle tubes instead of one thick needle tube, reduction of pain due to achievement of a smaller cross-sectional area of needle tubes is expected.

Meanwhile, recently, drug solutions having a high viscosity such as hyaluronic acid have also been used as an object to be injected. Since such a drug solution could not pass through thin needle tubes, a thick needle tube is used as before, and thus measures taken to reduce pain were insufficient.

In addition, such a drug solution is more difficult to be dispersed in intradermal tissue, and it has a tendency to remain in the injection site, i.e. a narrow area centering on the edge of the needle tube. Thus, dispersion of a drug solution in tissue was insufficient by the use of one needle tube.

Furthermore, an injection needle is mainly comprised of a needle tube and a hub that supports the base end side of the needle tube and connects to a syringe. This hub has been conventionally manufactured by an injection molding process of plastic, utilizing a single shaping mold. Accordingly, at the time of designing a needle base, it was impossible to choose to increase the size due to limitation by the unified standard of the size of a syringe connecting part.

Thus, even if spaces between injection sites are broadened in the type of injection needle that supports a plurality of needle tubes to cope with a drug solution that is difficult to be dispersed as stated above, there was a limitation in broadening the spaces between the needle tubes that are arranged in parallel.

In addition, approach to painlessness is an objective that is commonly required for an injection needle. Since a drug solution having a low viscosity can be injected even if the needle tube is made thin, it is expected that pain due to puncturing by a needle tube at the time of injection can be further reduced if the needle tube can be made thinner. In such a case, the total number of needle tubes would be increased. However, as stated above, there is a limitation in designing of the size of a needle base. Thus, there is also a limitation in increasing the number of needle tubes, as long as they are supported by the needle base.

### [Citation List]

### [Patent Literature]

Patent Literature 1: Japanese National Phase PCT Laid-open Publication No. 2013-539990

### [Summary of Invention]

### [Problems to be solved by the Invention]

The present invention is made by focusing on the above-described conventional problem, and the purpose thereof is to provide a novel and useful injection needle that allows design of the size of a needle base without being limited by the size of a syringe connecting part.

### [Means for solving the Problems]

In order to achieve the above-described objective, the first invention is an injection needle characterized in comprising: a plurality of needle tubes having a discharge opening for a drug solution in the needle tip; a hub that supports each of the plurality of needle tubes at the base end side and connects to a syringe; and a needle tube shield part that is attached to the base end side of the needle tube to shorten the length of the projected tip of the needle tube, wherein the hub is configured in a state divided into a needle base that is formed to have through holes into which the needle tubes are inserted, and a tubular housing where both ends in the axial direction are opened and the needle base is fitted and stored in the inside of one end in the axial direction, wherein the housing and the needle base are connected by an adhesive that infiltrates from the needle tip side into a fitting gap formed therebetween, or by welding, wherein the needle tube shield part is in a box-lid shape, and the back side of a ceiling part thereof is provided with guiding parts that surround the needle tubes for guiding, and wherein the guiding parts abut on the needle tubes projecting surfaces of the needle base, and the side part annularly holds and abuts on the housing.

The second invention is an injection needle defined by the first invention, characterized in that, when seen from the puncture/removal direction of the needle tube, the outline of the cross section of the needle base is positioned to the outer side than the inner outline of the cross section of the syringe connecting side of the housing.

The third invention is an injection needle defined by the first or second invention, characterized in that an opening on the needle tip side of a fitting gap is expanded by the retraction of the needle base side to form an annular concave part to act as an entrance to the fitting gap and a filling portion for an adhesive.

The fourth invention is an injection needle defined by any of the first to third inventions, characterized in that the side part of the needle tube shield part extends to the entry site of an adhesive or a melt fluid, which is generated at the time of welding, into the fitting gap for holding and abutting.

The fifth invention is an injection needle defined by any of the first to fourth inventions, characterized in that the guiding part is configured to be a tubular body.

The sixth invention is an injection needle defined by any of the first to fifth inventions, characterized in that the needle base and the housing are both injection molded parts.

### [Effect of the Invention]

According to the injection needle of the present invention, the size of a needle base can be designed without limitation of the size of a syringe connecting part, and by achieving a larger size of the needle base, it is possible to broaden the spaces between a plurality of needle tubes that are arranged in parallel, or increase the number of needle tubes.

In addition, the injection needle of the present invention can be manufactured by injection molding as in the case of a conventional one by utilizing two shaping molds.

### [Brief Description of the Drawings]

Figure **1** is an entire perspective view of a five-needle type injection needle defined by an embodiment of the present invention.
Figure **2** is an exploded perspective view of the injection needle of Figure **1****.**
Figure **3** is a halved perspective view of the injection needle of Figure **1****.**
Figure **4** is a side view of the injection needle of Figure **1****.**
Figure **5** is a cross-sectional view in the direction corresponding to Figure **4****.**
Figure **6** is a cross-sectional view in the vertically intersecting direction of Figure **5****.**
Figure **7** is an exemplary view of action of force that works at the time of injection, based on Figures **5** and **6****.**
Figure **8** is a view showing an exemplary modification of the needle base of the injection needle of Figure **1****.**
Figure **9** is an entire perspective view and a halved perspective view of a nine-needle type injection needle that is different from that of Figure **1****.**

### [Mode for carrying out the Invention]

An injection needle **1** defined by an embodiment of the present invention will be explained in accordance with the drawings.

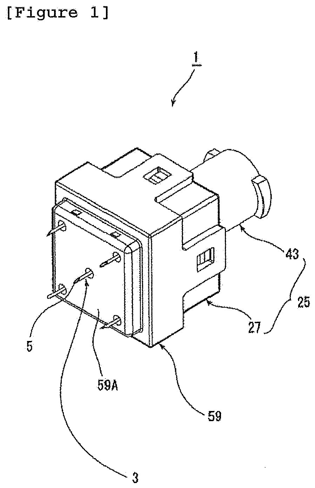

As shown in Figure **1****,** the injection needle **1** is a five-needle type, and it is provided with five needle tubes **3** having the same diameter and that are linear.

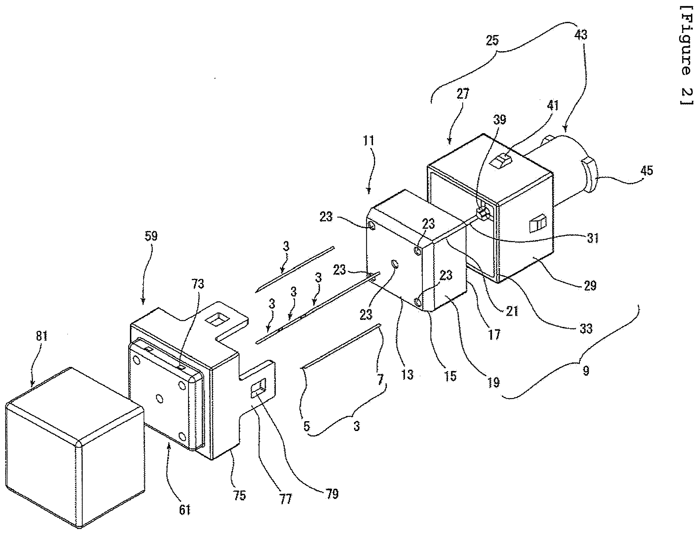

In each needle tube **3,** as shown in Figure **2****,** a needle tip **5** is formed as a discharge opening for a drug solution, and a base end **7** is supported by a hub **9** to be integrated. This hub **9** is comprised of two injection molded parts.

One of the injection molded parts is a needle base **11.** This needle base **11** is shaped in a block state that is substantially a square shape, and the square-shaped surface on the side to which the needle tip **5** of the needle tube **3** protrudes, is a front surface **13.** The whole circumference of the edge part of this front surface **13** is uniformly chamfered to from a inclined surface **15,** and the front surface **13** is in a similar scale down shape of a back surface **17** by being scaled down just as much as the chamfered portion. The front surface **13** and the back surface **17** are in parallel, and four side surfaces **19, 19,** ... are exposed in the vertically intersecting direction therebetween. The corner parts at the boundaries of the adjacent side surfaces **19, 19** are all provided with a roundness **21.**

From the front surface **13** to the back surface **17,** five through holes **23** which penetrate the needle base **11** are formed. Each through hole **23** linearly extends in the direction that vertically intersects with the front surface **13** and the back surface **17.** The cross-section surface in the vertical direction thereof is in a circular shape, and it has the same diameter except for the opening end which opens to the front surface **13.** Furthermore, the opening end side is expanded in a tapered state, and it is an entrance and a reservoir for an adhesive.

Accordingly, five through holes **23, 23,** ... are arranged in a state that they are parallel with one another, and among the five through holes **23, 23,** ... , one through hole is opened at the center position of the front surface **13,** and other four are opened at locations closer to four corner parts of the front surface **13.** Five through holes **23, 23,** ... each match the above-described five needle tubes **3,** and one needle tube **3** is inserted in one through hole **23.** The center of the through hole **23** opened in the center position is the center in the axial direction of the entire injection needle **1,** and this axial direction is the puncture/removal direction of the needle tube **3.**

Hereinafter, explanations will be made by utilizing this "axial direction" as the standard index of directions. In addition, one end of the axial direction will be expressed as the "puncture side of the axial direction", and the other end will be expressed as the "removal side of the axial direction".

The other of the injection molded parts is a housing **25.** This housing **25** is formed in a tubular shape, and the axial direction thereof matches with the center of the axial direction of the injection needle **1.** Both ends in the axial direction are opened.

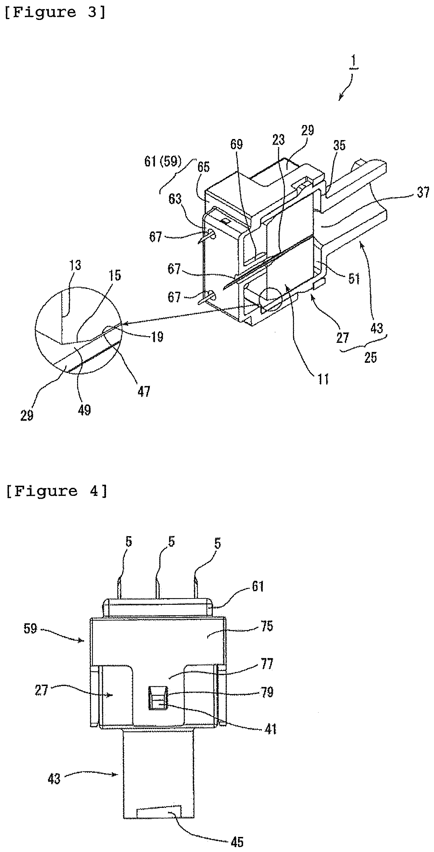

As shown in Figures **3** and **4****,** the puncture side of the axial direction is a fitting storage part **27** of the needle base **11.**

This fitting storage part **27** is configured in a hollow square shape by the connection of the thickness sides of four rectangular plate side parts **29, 29,** ..., and the vertical section in the axial direction, where the thickness sides can be seen, forms a substantially square frame. The corner part in the inner surface is provided with a rounded depression **31.** This frame size is the same size in any vertical section in the axial direction.

The edge on the puncture side of the axial direction of the fitting storage part **27** is chamfered. However, a flat edge surface **33** having a certain amount of area is left in the inward side. This edge surface **33** is on the vertical section in the axial direction.

As shown in Figure **3****,** from the edge on the removal side, a plate-like inward flange **35** inwardly extends and narrows the opening. A communication is established in the axial direction via a communication hole **37** in a circular shape, which is left at the center. In each of the corner parts at four corners blocked with the inward flange **35,** a stopping convex part **39** that is in a square shape is formed.

In addition, as shown in Figure **2****,** a hook projection **41** is formed on each of the external surfaces of the four side parts **29, 29,** ....

On the other hand, the removal side of the axial direction is a syringe connecting part **43.**

The vertical section in the axial direction of this syringe connecting part **43** is in a circular shape, and it is formed to have the same diameter along the axial direction. The inner diameter of the syringe connecting part **43** is substantially the same as the communication hole **37,** and this is continuous with the fitting storage part **27** by having the inward flange **35** as the stepped part.

The edge on the removal side is fully opened, and the outside surface thereof is formed to have a connecting convex part **45** for syringe connection.

The needle base **11** is fitted and stored in the internal space of the fitting storage part **27** in a nested-state, in a posture where the front surface **13** is exposed towards the puncture side of the axial direction. Thus, the roundness **21** in the corner part faces the depression **31** of the fitting storage part **27.**

The needle base **11** is loosely fit in the fitting storage part **27,** and as shown in Figure **3****,** although a fitting gap **47** is formed between the side surface **19** of the needle base 11 and the inner surface of the side part **29** of the fitting storage part **27,** and also between the roundness **21** and the depression **31,** this is a slight gap. Thus, a relative rotation around the axis is not possible.

Furthermore, an expanded annular concave part **49** is formed between the inclined surface **15** and the inner surface of the side part **29** on the fitting storage part **27** side. The annular concave part **49** is formed such that the vertical section in the axial direction is in a triangular shape, and it is continuous with the fitting gap **47.**

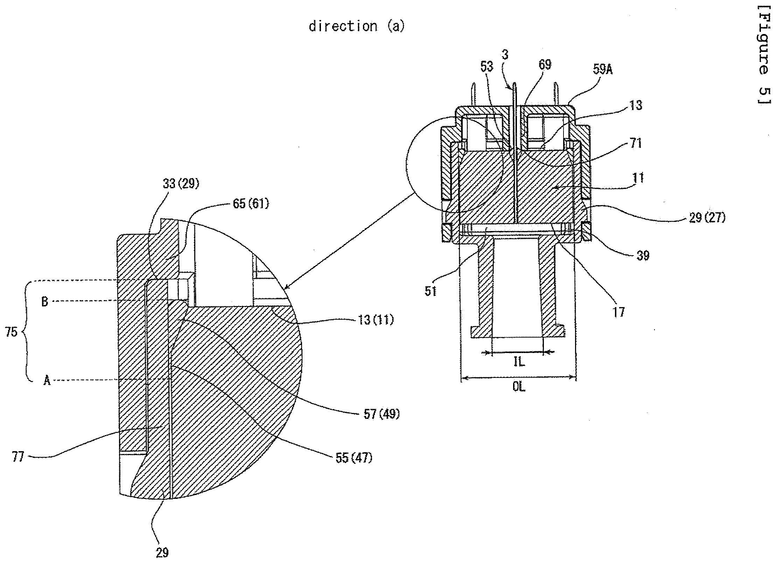

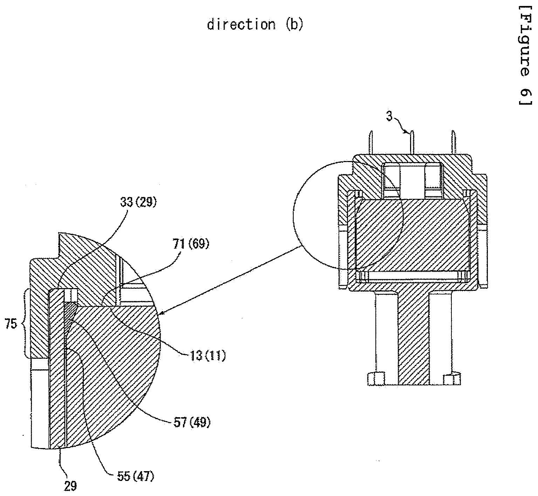

As shown in Figures **5** and **6****,** the length of the fitting storage part **27** in the axial direction is designed to be longer than the length of the needle base **11** in the axial direction, and the back surface **17** of the needle base **11** is embedded to a depth that abuts on the stopping convex part **39** (Figure **2**) to be stopped there. Accordingly, a space **51** for guiding a drug solution is left between the back surface **17** and the inward flange **35.** This space **51** is broadened in the radially outward direction more than the flange connecting part **43,** and the broadened site is a portion that becomes undercut when the needle base **11** and the fitting storage part **27** are regarded as a single injection molded part.

On the other hand, the front surface **13** of the needle base **11** is in a state that is one stage lower than the edge surface **33** of the fitting storage part **27.**

Reference numerals **55, 57** show joint parts that are fixed. As the joining technique, not only adhesion by an adhesive, but so-called welding is also applicable. The joint parts **55, 57** are comprised of such adhesive.

The curing type of the adhesive can be a room temperature curing adhesive, a thermosetting adhesive, or UV-curable adhesive.

However, the joint part is preferably a thermosetting adhesive or UV-curable adhesive.

These adhesives have been conventionally used for adhesion and fixation of a needle tube to a hub. The adhering part **53** is interposed for adhesion of the needle tube **3,** which is inserted in the through hole **23** of the needle base **11,** against the needle base **11.** The adhesive is filled by having the opening end side of the through hole **23** as its entrance, and the adhesive deeply penetrates into the gap between the needle tube **3** and the through hole **23** by capillary action. The opening end side eventually becomes a reservoir where the adhesive is swelled in a bump state and accumulated.

The needle tube **3** that is used, is sufficiently long. Thus, when filling an adhesive, it is easy to support the needle tube **3** in an inserted state, and the adhesive will not touch the needle tip **5.**

The adhering part **55** is interposed for adhesion and fixation of the needle base **11** against the fitting storage part **27.** The annular concave part **49** is an entrance of an adhesive, wherein the adhesive infiltrates into the fitting gap **47** and is solidified. The depth of penetration of an adhesive into the fitting gap **47** exceeds half the length of the needle base **11,** and the end thereof is point "A". In addition, an adhesive is swelled in a bump state at the annular concave part **49,** and the end thereof is point "B". The adhering part extends from the fitting gap **47** to the annular concave part **49,** and it is interposed between the needle base **11,** which is the inward side, and the fitting storage part **27,** which is the outward side, in an inner tube state to adhere the two with a large adhesive area.

For convenience of explanation, the joint part may be distinguished between the joint part **55** on the fitting gap **47** side and the joint part **57** on the annular concave part **49** side. However, a seamless and continuous joint part is formed, and the flow line is in succession.

As stated above, the needle tubes **3, 3,** ... are fixed in a manner that the base end **7** is supported against the needle base **11,** and the needle base **11** is fixed against the housing **25** in the above-described manner to be integrated. Furthermore, as shown in Figure **1****,** a needle tube shield part **59** is attached to the needle base **11** side.

This needle tube shield part **59** is also an injection molded part, and it has a box-lid shape.

As shown in Figures **2** and **3****,** a ceiling part **61** is in the form of a plate having a square shape, and has a two-stage structure where a four-side peripheral part **65** is made one stage lower with respect to a central part **63.** This central part **63** is formed to have five through holes **67, 67,** ... where the cross section is a circular shape, and a tubular guiding part **69** is continued at the back surface along the hole edge of each through hole **67.** This tubular guiding part **69** has the same diameter and is linear.

Among the five through holes **67, 67,** ..., one through hole is formed at the center position of the central part **63,** and other four are each formed closer to the four corner parts of the central part **63,** as in the case of the through hole **23** on the front surface **13** side of the needle base **11.** As shown in Figures **5** and **6****,** each tubular guiding part **69** extends beyond the ceiling part **61** which has a step, and the edge surface **71** is a flat surface that is in parallel with the plate surface of the ceiling part **61.**

In addition, as shown in Figure **2****,** on the outer surface of the ceiling part **61,** pressing convex parts **73, 73** in pairs are provided in two opposing surfaces, and each pressing convex part **73** is formed in a state that is outwardly projected.

With respect to the ceiling part **61,** four plate-like side parts **75, 75,** ... are vertically raised, and tongue pieces **77, 77,** ... are created to extend from each of them. Each tongue piece **77** extends from the central portion of the end edge of the side part **75,** and a hooking hole **79** in a square shape is formed therein.

As shown in Figure **4****,** the needle tube shield part **59** is covered on the square-shaped fitting storage part **27** as a square-shaped box lid to be integrated, and the side parts **75, 75,** ... surround the side parts **29, 29,** ... of the fitting storage part **27** from the outside, thereby allowing the hook projection **41** on the fitting storage part **27** side to be hooked and locked in the hooking hole **79** of the needle tube shield part **59** side. Accordingly, relative movements of the needle tube shield part **59** in the axial direction and the direction around the axis with respect to the fitting storage part **27** (= housing **25**) are restricted.

In this covered state, one tubular guiding part 69 corresponds to the axial direction of one needle tube 3, and the base end **7** side of the needle tubes **3, 3,** ... is shielded by the needle tube shield part **59,** thereby allowing the length of the projected tip to be short. As stated above, the needle tube **3** projects from the front surface **13** of the needle base **11** by a certain length such that the operation is facilitated when fixing to the needle base **11** with an adhesive. In this regard, this needle tube shield part **59** is utilized and the front surface of the needle tube shield part **59** is set as a reference plane **59A** of puncture depth as shown in Figures **5** and **6****,** to allow accurate injection to a predetermined depth in tissue.

In addition, in this covered state, the peripheral part **65** of the ceiling part **61** abuts on the edge surfaces **31, 31,** ... of the side parts **29, 29,** ... of the fitting storage part **27,** and the side part **75** abuts and holds from the outside. The side part **75** has a certain length, and it extends to around point A of the adhering part 55 entered into the fitting gap **47.**

In addition, the edge surface **71** of each tubular guiding part **69** abuts on the front surface **13** of the needle base 11. One edge surface **71** is positioned at the center of the front surface **13,** and four edge surfaces **71** are uniformly disposed in the surrounding of the front surface **13.** Since the edge surface **71** closer to the corner part does not span the inclined surface **15** of the needle base **11,** it does not contact the adhering part **57** which is in a bump state, and the entirety thereof abuts on the front surface **13.**

The injection needle **1** of the present invention is configured in the above-described manner, and when seen from the puncture/removal direction of the needle tube **3,** the outline (OL) of the cross section of the needle base **11** is positioned to the outer side than the inner outline (IL) of the cross section of the syringe connecting side of the housing **25.**

In other words, the size of the needle base **11** is increased without being limited by the size of the syringe connecting part, and five needle tubes **3** are disposed with a sufficient space between each other, on the front surface **13** of the needle base **11.**

Furthermore, as shown in Figure **2****,** at the time of storage, a cap **81** is covered on the needle tip **5** side of the needle tube **3.** The pressing convex parts **73, 73** made in the ceiling part **61** are pressed by the inner surface of the cap **81** to be closed.

The length of the projected tip of the needle tube **3,** i.e. , the length until the needle tip **5** of the needle tube **3** by setting the exposed surface of the ceiling part **61** of the needle tube shield part **59** as a reference, is from 0.05 to 3.5 mm.

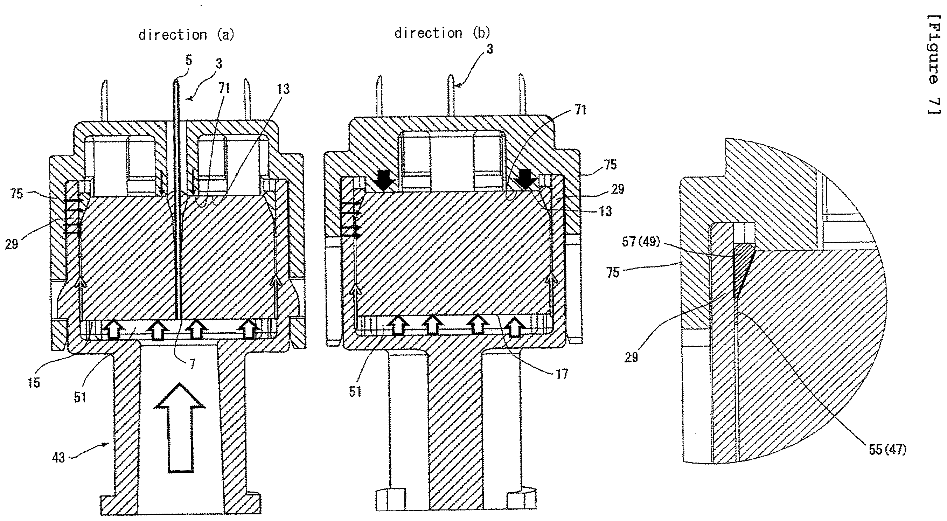

The influence of pumping of a drug solution at the time of injection is shown in Figure **7****.**

At the time of injection, when a drug solution is infused to the syringe connected to the injection needle **1** and is pressed by the plunger, the drug solution is pumped from the syringe connecting part **43** of the housing **25** towards the fitting storage part **27.**

Furthermore, when the drug solution reaches each of the base ends **7, 7,** ... of five needle tubes **3, 3,** ... , it enters into each of them to be discharged from the needle tips **5, 5,** ....

The drug solution firstly enters into the space **51** from the syringe connecting part **43,** and it is distributed and fed to each needle tube **3** therefrom upon receiving pressure. Thus, as shown in white arrows, the drug solution reached around the back surface **17** side of the needle base **11** tries to push up the needle base **11** from the fitting storage part **27.**

In contrast, since the edge surface **71** of the tubular guiding part **69** of the needle tube shield part **59** abuts on the front surface **13** of the needle base **11,** it tries to push back as shown in black arrows. Since the edge surfaces **71, 71,** ... are uniformly disposed in the center of the axis and the surrounding thereof, the power is balanced in the axial direction, and no moment is generated.

In addition, the adhering part **57** which is buried in the annular concave part **49** is in a bump state and adheres to the inclined surface **15** of the needle base **11** so as to increase the adhesive area. At the same time, as shown in the black arrows, the side part **75** of the ceiling part **61** holds the side part **29** of the fitting storage part **27** to apply force that suppresses swelling to the outside. Thus, the adhesive strength of the thick-line portion shown in the enlarged view is effectively reinforced. Accordingly, this adhering part **57** also cooperates to effectively resist the above-described pressure to push up.

In addition, the drug solution that reached around the fitting gap **47** tries to cleave the adhering part **55** filled in the fitting gap **47** between the side part **29** of the fitting storage part **27** and the needle base **11.** However, also regarding the fitting gap **47,** as shown in the black arrows, the side part **75** of the ceiling part **61** holds the side part **29** of the fitting storage part **27** to apply force that suppresses swelling to the outside. Further, the above-described adhering part **57** exerts a strong damming effect. Accordingly, the above-described pressure to cleavage is effectively resisted.

In the present invention, when a drug solution is pumped, the needle base **11** receives pressure in the direction to be pushed out from the fitting storage part **27.** However, as stated above, by devising the structures of the needle base **11,** fitting storage part **27,** and needle tube shield part **59,** and the assembled state thereof, the relative position of the fitting storage part **27** to the needle base **11** is accurately maintained, and a pressure drop during injection is prevented.

In addition, the guiding part of the needle tube **3** is formed to have a tubular structure in the needle tube shield part **59,** thereby achieving weight reduction.

The drug solution used in the injection needle **1** of the present invention is typically a solution, gel or suspension that contains the drug solution. Drugs that can be used are substantially not limited as long as they are not such drugs unsuitable for transdermal administration.

Major drugs can be, for example, hyaluronic acid, collagen, botox or the like, antibacterial drug, antiviral drug, vaccine, antineoplastic drug, immunosuppressant, steroid drug, antiinflammatory drug, antirheumatic drug, arthritis therapeutic agent, antihistaminic drug, antiallergic drug, antidiabetic drug, hormone drug, bone/calcium metabolic drug, vitamin, blood preparation, hematopoietic drug, antithrombotic drug, antihyperlipidemic drug, antiarrhythmic drug, vasodilator, prostaglandin, calcium antagonist, ACE inhibitor, β-blocked, antihypertensive drug, diuretic drug, xanthine derivative, β agonist, antiasthmatic drug, antitussive drug, expectorant drug, anticholinergic drug, antidiarrheal drug, stomachic digestant, antiulcer drug, purgative drug, sleeping pill, sedative, antipyretic, cold medicine, antiepileptic drug, antipsychotic drug, antidepressant drug, antianxiety drug, central nervous system stimulant, parasympathomimetic drug, sympathomimetic drug, antiemetic drug, central stimulant, antiparkinsonian drug, muscle relaxant drug, anticonvulsant, anesthetic, antipruritic drug, antimigraine drug, diagnostic agent, oligonucleotide, genetic drug, and the like.

However, the drugs are preferably proteins, peptides, polysaccharides, oligonucleotides, DNAs and the like that do not exert effects or that are weakened in oral administration. Specifically, the drugs are high-molecular weight pharmaceutical products such as insulin, growth hormone, interferon, calcitonin and the like.

The embodiments of the present invention have been explained above. However, the specific configurations of the present invention are not limited to the above-described embodiments, and design changes that do not deviate from the gist of the present invention are also included in the present invention.

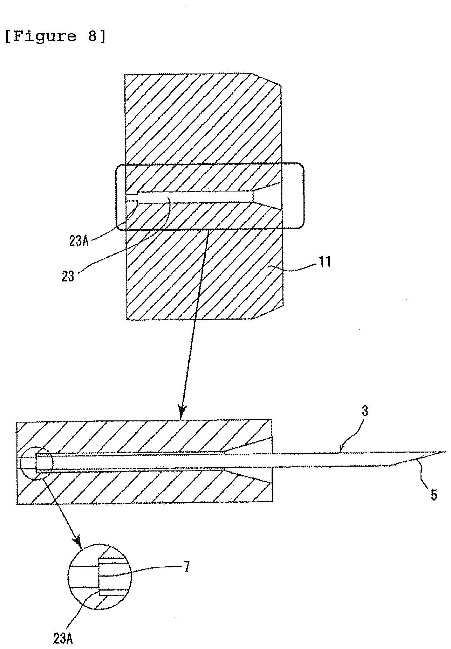

For example, as shown in Figure **8****,** a step **23A** can be provided in the through hole **23** of the needle base **11** so that the base end **7** of the needle tube **3** is stopped at that step **23A.**

In addition, since the reference plane **59A** of the needle tube shield part **59** is made large, it can be considered that this could be curved along the shape of the surface of the site to be injected.

Furthermore, although the front surface **13** of the needle base **11** has a square shape, it can be made into a circular shape and the like.

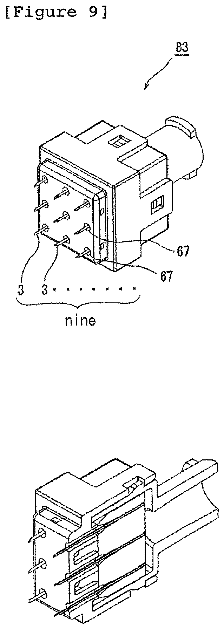

Figure **9** shows an injection needle **83,** which is another example. This injection needle **83** is a nine-needle type. In this manner, the size of the needle base can be designed without being limited by the size of the syringe connecting part. Thus, it is possible to increase the number of needle tube **3** than the conventional ones.

In addition, it is also possible to widen the pitch of the needle tube **3,** and the number of the needle tube **3** can be increased from 2 to 36 needle tubes.

In addition, not only adhesives, but a welding technique can also be utilized for the joining parts **55, 57.** In that case, the fitting gap **47** is filled with a fluid entered from the needle base **11** and the fitting storage part **27** surrounding the fitting gap **47** by melt flow, and a matter corresponding to the adhering part **55** is formed. Further, although the annular concave part **49** is generally not formed on the opening end side, a matter corresponding to the adhering part **57** is formed after welding. Accordingly, the same effect as in the case of using an adhesive can be enjoyed.

The types of welding can be laser welding, ultrasonic welding, vibration welding, and heat welding.

### [Reference Numeral List]

- **1**: injection needle (five needles)
- **3**: needle tube
- **5**: needle tip
- **7**: base end
- **9**: hub
- **11**: needle base
- **13**: front surface
- **15**: inclined surface
- **17**: back surface
- **19**: side surface
- **21**: roundness
- **23**: through hole
- **25**: housing
- **27**: fitting storage part
- **29**: side part
- **31**: depression
- **33**: edge surface
- **35**: inward flange
- **37**: communication hole
- **39**: stopping convex part
- **41**: hook projection
- **43**: syringe connecting part
- **45**: connecting convex part
- **47**: fitting gap
- **49**: annular concave part
- **51**: space for guiding a drug solution
- **53**: adhering part
- **55, 57**: adhering part
- **59**: needle tube shield part
- **61**: ceiling part
- **63**: central part
- **65**: peripheral part
- **67**: through hole
- **69**: tubular guiding part
- **71**: edge surface
- **73**: pressing convex part
- **75**: side part
- **77**: tongue piece
- **79**: hooking hole
- **81**: cap
- **83**: injection needle (nine needles)

## Claims

1. An injection needle comprising a plurality of needle tubes and a hub for supporting the plurality of needle tubes, wherein:
the hub comprises a needle base having a plurality of through holes into which the plurality of needle tubes are inserted and a housing for storing the needle base, wherein the needle base and the housing are joined;
the housing comprises a syringe connecting part on the base end side of the housing, and the syringe connecting part is configured to be connected to a syringe; and
a cross-sectional area of the needle base is larger than a cross-sectional area of the syringe connecting part.

2. The injection needle according to claim 1, wherein:
the injection needle further comprises a needle tube shield part;
the needle tube shield part comprises a ceiling part and a side part;
the plurality of needle tubes are disposed to be projected from the ceiling part of the needle tube shield part; and
the needle tube shield part and the housing are configured such that an inner surface of the side part of the needle tube shield part abuts on an outer surface of the housing.

3. The injection needle according to claim 1, wherein a gap is formed between the needle base and the housing, and the gap is filled with an adhesive or melt fluid.

4. The injection needle according to claim 2, wherein a gap is formed between the needle base and the housing, and the gap is filled with an adhesive or melt fluid.

5. The injection needle according to claim 4, wherein a range where the inner surface of the side part of the needle tube shield part abuts on the outer surface of the housing includes a range where the gap is filled with the adhesive or melt fluid.

6. The injection needle according to claim 2, wherein the needle tube shield part further comprises a plurality of guiding parts for guiding the plurality of needle tubes, and the plurality of guiding parts abut on the front surface of the needle base.

7. The injection needle according to claim 3, wherein: an annular concave part is formed in an outer edge of a tip part of the needle base; an additional gap is formed between the needle base and the housing by the concave part; and the additional gap is further filled with an adhesive or melt fluid.
